# EUROPEAN PATENT SPECIFICATION

(11) **EP 1 633 483 B1**
(45) Date of publication and mention of the grant of the patent: **06.05.2009**
(21) Application number: 04736081.3
(22) Date of filing: 04.06.2004
(51) Int. Cl.: B01L 9/00, G01N 35/10, B65G 1/04, B01L 3/00

(54) **BIOLOGICAL APPARATUS**
BIOLOGISCHE VORRICHTUNG
APPAREIL BIOLOGIQUE

(30) Priority: 04.06.2003 GB 0312800
(43) Date of publication of application: 15.03.2006
(73) Proprietor: Genial Genetic Solutions Limited, Champions Business Park Arrowebrook Road, Upton Wirral, CH49 0AB (GB)
(72) Inventor: CREES, Lawrence, Carl, Runcorn, Cheshire WA7 4QX (GB); CREES, Jeremy, Carl, George, Runcorn, Cheshire, WA7 4QX (GB)
(74) Representative: W.P. Thompson & Co.
(86) International application number: PCT/GB2004/002379
(87) International publication number: WO 2004/108289

(56) References cited:
- WO-A-87/00086
- US-A- 4 065 359
- US-A1- 2002 114 733
- DATABASE WPI Section EI, Week 197529 Derwent Publications Ltd., London, GB; Class S03, AN 1975-H4713W XP002296572 & SU 440 578 A (BLINOV V A) 5 February 1975 (1975-02-05)

## Description

The present invention relates to an apparatus for the processing of biological material comprising a plurality of rotatable platforms that have one or more samples of biological material disposed thereon.

Advances in biochemistry and in particularly molecular biology and cytogenetics have resulted in reliable tests for certain types of diseases or conditions. Many tests require long and complex protocols and often require certain steps to be adhered to in a time dependant manner. Most tests are performed on patient samples, such as biopsies, blood samples and other tissue samples for example. These samples need to be processed prior to analysis or further processing. Therefore, much of the time spent by clinical scientists is in processing samples, rather than analyzing the processed samples and producing a report of the results for the physician accurately to diagnose a disease or medical condition. Indeed, as such processing can be quite tedious and requires a high degree of skill, there is the risk that a sample may be processed incorrectly, producing a wrong result which may have serious consequences for an individual. Furthermore, a number of protocols involve hazardous chemical agents that necessitate the protocol being conducted in fume hoods/cabinets or require specialist protective clothing to be worn.

Common tests performed by clinical scientists include karyotyping of individuals for predisposition to diseases or testing whether an individual condition is related to a chromosomal abnormality such as Down's syndrome. Immunostaining can show a range of conditions in cell samples such as excess endothelial cell growth in tumour samples. Fluorescent *in situ* hybridisation (FISH) and *in situ* hybridisation (ISH) can be used for assessing over expression of genes or proteins in cells. There are many more tests (and variations of those discussed) that are available for testing samples and these will be apparent to one skilled in the art. Most of these tests require some type of cell fixing, in order to allow ligands to be bound to the surface of a cell (or organelle) or to fix the cell onto the surface of a slide. Such fixing by its very nature often requires hazardous chemicals such as cross-linking agents or organic solvents that pose a health risk to the clinical scientist.

In order to address some of these problems outlined above and to use the clinical scientists time more effectively, a number of automated machines for processing of samples have been produced.

A flat bed apparatus is commonly found in laboratories and comprises a plurality of wells disposed on a flat horizontal bed. Reagents are then dispensed via a single or multibarrelled tip that is linked to a motorized platform that moves in the X and Y planes servicing all the wells. The single tip often utilizes a vessel with water for cleaning the tip in between reagent additions. Such apparatus are large and take up vital bench space in the laboratory. Due to the time dependency of some steps in certain protocols, the number of wells which can be serviced can be limited by the use of one or two tips (especially if the tip requires a washing step after every reagent is dispensed or aspiration performed at each well position). There is also a tendency for the tips to become contaminated over prolonged use and tips are often prone to blocking, rendering the apparatus unusable until it is serviced. As this apparatus usually requires hazardous chemical reagents, it is often placed in a fume hood, again taking up essential experimental work space in the laboratory.

US 5,976,871 discloses a cytogenetic chamber which includes a chamber enclosure, a drying cavity in the enclosure, a door and a hand-insertion port through the door which can maintain a substantially uniform air flow for drying of the processed material. This device is not automated and requires material to be processed by hand.

Both WO 91/13335 and US2002/0114733 discuss an immunostaining apparatus for preparing slides, having a reagent application zone and a reagent supply zone, wherein the apparatus has a carousel with two discs, one disc being the application zone the other disc being the reagent supply zone. Such an apparatus has the disadvantage that each sample is treated individually and therefore the number of samples that may be processed will be limited, should a protocol be time dependant. There is also the problem of fumes from the reagents diffusing into the surrounding area, so such an apparatus would need to be placed in a fume hood. Furthermore, the reagents need to be loaded onto the reagent supply zone manually which will be time consuming.

It is an object of the present invention to address one or more of the problems associated with the prior art apparatus and methods of processing biological samples. It is also an object of the present invention to provide an apparatus for processing biological material that can simultaneously process multiple samples, which would be particularly advantageous should the process to be performed on samples having time dependant protocols. Furthermore, it is also an object of the present invention to provide an apparatus that can be used in confined spaces and need not be placed in a fume hood.

According to the present invention, there is provided an apparatus for processing biological materials, the apparatus comprising a plurality of stacked, rotatable platforms, each platform having a plurality of sample receiving areas located thereon, the apparatus having a first dispensing member mounted for dispensing reagent and/or a sample to a sample receiving area on a first stacked platform and a second dispensing member mounted for dispensing reagent and/or a sample to a sample receiving area on a second stacked platform, each platform being rotatable to move sequential sample receiving areas thereon into orientation with the mounted reagent dispensing member to receive reagent therefrom.

The apparatus may further comprise a third dispensing member mounted for dispensing reagent and/or a sample to a sample receiving area on a third stacked platform. Each stacked platform may have an associated dispensing member mounted for dispensing reagent and/or a sample to a sample receiving area on each stacked platform. Preferably, the apparatus further comprises a removing member for removing waste reagent from the sample receiving area. It will be evident that the apparatus need not be limited to predefined number of dispensing and/or removing members due as the number will depend upon the number of stacked platforms in addition to the protocol being applied to the samples and whether separate members are required for different processing steps.

The apparatus for processing biological material comprises a plurality of rotatable platforms, each platform capable of receiving one or more samples of biological material, the sample being disposed on a holding means, the apparatus further comprising a dispensing and removal means capable of dispensing one or more reagents and/or samples to the sample receiving area and removing waste products from the sample.

The present invention allows multiple samples to be processed at the same time and furthermore, due to the platform arrangement of the apparatus it occupies less space than prior art devices. Furthermore, the present invention also reduces manual operator variability and allows trained and skilled operators to be utilized more effectively in the laboratory.

The term "platform" encompasses a number of different structures such as discs or armed members and indeed any structures that would allow the apparatus to operate effectively. The sample receiving areas may be removable so that the sample is loaded outside of the apparatus.

The apparatus may be substantially contained within a releasably sealed housing. Therefore, a number of parameters can be controlled within the housing when processing is taking place. For example, the atmosphere may be controlled in order to allow for correct drying of the sample if necessary either between steps in a protocol, or at the end of processing the material. The atmosphere may be adapted to counteract the ambient temperature outside of the apparatus and indeed, the atmosphere of the apparatus may be controlled in such a manner that local environments near to or around a sample or platform of samples may be controlled. In order to control local environments, a given platforms or the atmosphere of the whole apparatus may be controlled by one or more of the following variables: temperature, relative humidity and volumetric air flow rate. These variables may be precisely controlled at the sensitive areas where the samples are held. Such control over the local environments or atmosphere may be used to dry samples (located on a cover slip or slide for example) after processing or in between steps or during the addition/removal of reagents. The control of local environments may utilize an air conditioning means disposed near to or in conjunction with the dispensing member and/or the removing member so that individual samples can be subjected to the required local environmental conditions of a given protocol. It will be apparent to one skilled in the art that all of the variables can be controlled by currently available air conditioning apparatus (albeit a small scale apparatus) and the conditioned air can be supplied to the samples, platforms or housing interior by pipes with baffles or similar diffusion devices if required. The air conditioning (including the drying of samples) may be automated and/or connected to a central processing unit. In order to assist in the automation of the air conditioning, sensors may used to relay conditional data to the central processing unit to adjust the variables where appropriate. The central processing unit may adjust the variables depending on the protocol used and/or may be adjusted for bespoke operations or protocols. Additionally, or alternatively, the sensor may comprise a turbidity monitor which is incorporated into the apparatus so as to assess a sample or batch of samples to ensure that all are subjected to the correct atmosphere (for drying a sample after the addition of a reagent). Furthermore, the housing will also allow any fumes from reagents to be contained substantially within the apparatus. The housing may also be connected to an air extraction means in order to remove fumes from the apparatus to an external source. The air extraction means may also comprise a fume treatment device located inside or on top of the apparatus, commonly such devices are known as extraction and scrubbing devices. The fume treatment device may be an extraction fan which passes fumes over a fume absorbent material before allowing the treated air to be expelled into the surrounding environment and/or for further treatment. The fume absorbent material may be a catalyst or a material (such as a column of soda lime for example) which absorbs unwanted compounds such as organic vapors. The treated air may also be recycled back into the apparatus and used for air conditioning to assist drying for example and thus reducing the overall power input requirement of the apparatus. The reagents may also be held within the housing to allow for the apparatus to be a complete processing unit. Access to the reagents may be made via a separate door disposed on the housing in order to allow for replacement and washing of the apparatus.

The platforms may rotate about a common central axis and may either rotate in unison or independently to one another. Preferably, the platforms are substantially horizontal and stacked above one another in a vertical manner. The platforms may also be of similar dimensions. The platform may also have one or more slots disposed from the edge of the platform to the centre of the platform to permit the platform to be removed and placed about the axis easily. It will be apparent that the use of slots in platforms will allow samples to be loaded quickly outside of the apparatus and also allow the apparatus to be cleaned/serviced on a regular basis. The slot may allow the platform to clip onto the axis, or placed on a sleeve that releasably engages the platform with the axis. Preferably, the slot will allow the platform to be located at a given position on the axis so that the samples are in the same location relative to the axis when the platform is placed back on the axis after removal. The platform may be substantially circular or octagonal in shape, although it could potentially be any shape. The samples will preferably be placed on standard laboratory equipment such as a petri dish or similar device. The petri dish (or similar device) will preferably be placed in the sample receiving area. The biological material may be placed on a slide or a slide cover slip and then placed onto a petri dish or similar dish. The sample receiving area may be angled relative to said platform in the range of 2° to 25° to allow liquid to collect at one part of sample. Preferably, the sample receiving area is angled in the range of 2° to 8°. The apparatus may contain any number of platforms and the exact number will depend on the size of apparatus required and the number of samples that require processing simultaneously. Each said platform may be manufactured from aluminium, stainless steel or polypropylene, although most hard and rigid materials that are capable of resisting degradation by harsh chemical agents may also be suitable.

Furthermore, the dispensing or removing member may comprise one or more tubes. Preferably, each tube is manufactured from aluminium, and optionally have a PTFE coated interior. The PTFE coating prevents any corrosive reagents from degrading the aluminuim tubing. Alternatively, a PTFE tube may be disposed within an aluminium tube. Other materials or alloys may be employed in the manufacture of the tubing. The dispensing and removing member can move vertically relative to the sample receiving reagents. Preferably, movement is afforded by the dispensing and removing members rocking on a pivot in order to allow the dispensing and/or dispensing members to touch or come into close contact with the sample when the biological material is situated underneath the dispensing and/or removing members. Alternatively, movement may be afforded by the dispensing and removing members moving on a vertical rod. The vertical movement allows the dispensing and/or removing member to be lifted from the sample in order to allow the platform to rotate so that the a further sample can be processed. When the new sample is in place the dispensing and/or removing member can touch or come into close contact with the sample.

Preferably, a separate tube is provided to each reagent. It will be evident to one skilled in the art that a number of reagents may be used in the apparatus and the specific reagents will be dependant upon the protocol being implemented on the biological material. For example, in the case of processing the biological material for cytogenetic analysis, you may utilize 3 reagents: a pre-hypotonic reagent such as potassium chloride (or a standard hypotonic reagent); water to wash the sample (in between addition and removal of reagents) and a fixative such as a methanol acetic acid solution (normally in a 3:1 ratio of methanol to acetic acid, however, if a differed ratio is required the apparatus can accommodate this and mix the fixative to the required concentration as required). A given reagent may be previously mixed or may be prepared within the apparatus prior to being applied to the biological material, the latter being the case with some reagents that do not have a long shelf life. For example, should the samples be subjected to a fixation protocol, the fixative may be mixed by the apparatus for dispensing to the sample. Preferably, the required fixative is automatically mixed (in the appropriate ratios and quantities) depending on the sample size and type and thereby processing the samples accurately and reducing cytoplasm residues in the sample for example. The constituents of the fixative may be defined by the user of the apparatus or automatically selected by the apparatus depending on the protocol being utilized. Furthermore, the reagents may be chilled prior to dispensing so as to widen. There may be multiple washing steps in order to gradually elute a reagent from a sample. For example, multiple washing steps may be required to remove all growth media prior to applying a fixative reagent to the sample. Similarly, multiple washing steps may be required to elute neat fixative from the sample. The reagents may be dispensed and/or the waste material removed by means of peristaltic pumps. Such pumps will preferably be electronically controlled so as to ensure the correct quantities of reagent are dispensed. Preferably, the dispensing members are capable of permitting a single controlled drop of a reagent of a known quantity onto the sample. To facilitate the control of the drop addition of a reagent, the dispensing member may rock about an axis to assist the drop from falling or be tapped mechanically to permit the drop to fall from the dispensing member. Alternatively, the apparatus may automatically reverse the direction of the dispensing pump after dispensing the required volume of reagent and in doing so, ensuring that "hanging drops" are not carried through into the next sample. The removing member may comprise two or more tubes. This is advantageous, as a number of prior art devices only have one tube that may become blocked easily due to contaminants (and often fragments of glass from broken cover slips) or fragments of samples that lodge in the tubing. Providing two tubes allows one tube to become blocked without preventing the apparatus from working. The removing member may be connected to a waste tank located within the apparatus or connected to waste disposal device located outside the apparatus. Should the waste tank become full to capacity or becomes near to full capacity, an alarm may sound so as to alert the user to the fact. Alternatively, or additionally, an indicator relating to the quantity of waste material in the tank may be provided. It will also be obvious to one skilled in the art that should certain reagents be incompatible with one another (such that they react adversely together), separate waste tanks may be required.

The apparatus may also have adjustable feet in order to allow the apparatus to be adjusted to suit different surface. Such feet allow small variations in levels or angles of surfaces to be overcome so that the apparatus can operate in the correct manner. Preferably a measuring device is also provided with the apparatus in order that the correct level of the apparatus can be adjusted by the user. Preferably, the measuring device comprises a spirit level or similar device. Alternatively, an electronic device which can determine the level of the apparatus is provided. The apparatus may additionally comprise a means by which the level of the feet are automatically adjusted. Preferably, there are 4 feet disposed at the 4 corners of the apparatus.

The apparatus may further comprise a sensor for detecting the presence of a sample and/or identifying the sample. Preferably, the sensor is an optical sensor. Alternatively, the sensor may be a magnetic sensor. It will be apparent to one skilled in the art that a number of sensors may be utilized in the present apparatus, such as a reflective optical sensor or a scanning laser sensor to name a few. The sensor may be provided in order to detect the characteristics of the processed biological material. It will be apparent that this type of sensor would be particularly suited to biological material that is processed in order to identify a result shown by a reporter molecule attached to a fluorescence marker, such as utilized in FISH or oligonucleotide reporter sequences. Although it is also envisaged that such a sensor may also comprise a microscope or an imaging device for post processing analysis of the sample. Supporting equipment required for the sensor, such as lighting requirements may also be provided and in order to facilitate imaging and/or the activity of the sensor, the housing can be made substantially sealed from external light.

The samples may have an identification means disposed thereon to assist the user in identifying the correct sample. Such an identification means may comprise a bar code, a dot code or a radio frequency microcircuit that uniquely identifies a sample. The identification means may be placed on the petri dish that the sample is placed upon, or on the slide or slide cover slip. The identification means may be used in conjunction with the senor as outlined above, by it may also be used in conjunction with an identification sensor to identifying the sample both inside and outside of the apparatus. A suitable sensor may be a laser scanner in the case of a bar code, or a radio frequency scanner in the case of radio frequency emitter for example. It will be apparent to one skilled in the art that the use of the identification means in addition to the a sensor for analyzing results will permit rapid and automated collation of data for a number of samples which can be printed off or linked to a sample database such as a Laboratory Information Management System (LIMS) for archiving and/or further data analysis. Therefore the apparatus will greatly reduce the time and requirement of repetitive tasks undertaken by the clinical scientist to perform the processing and analysis of biological samples.

The apparatus may further comprise a proprietary easy-dry slide based mechanically-secured culture chamber for use both internally to the apparatus and externally. The culture chambers may be used in conjunction with the identification means and enables culturing , drying and processing of surface cultures to be performed on a slide rather than a cover-slip. The culture chamber allows for in-situ (surface) cultures for cytogenetic analysis to be grown on slides, rather than cover slips, thus supporting unique labelling of each sample by bar coding etc employed directly on the slide. Furthermore, as the chamber itself is mechanically clamped onto the slide itself, upon manual or automated removal for post-culture processing, removal of the seal is in its entirety, unlike all current "stuck on/sealed on" slide-mounted chambers which leave a residue on the slide, preventing even evaporation of fix from the slide and hence preventing their usage for cytogenetic slide drying.

The apparatus may also comprise a UV light source that is used to treat the samples. For example, the UV light source may be used in the procedure for chromosome banding or for cross-linking reagents. If the UV light source is used during chromosome banding or for cross-linking reagents or proteins for example, and it may be used in conjunction with the air conditioning means, which in turn may or may not be automated or programmed into the apparatus. It will be evident that the UV light source may be applied to the whole of the housing or applied to individual platforms or samples. The UV light source may be applied by means of mirrors or splitters so that that multiple UV light streams can be produced from a small number or UV light sources. Preferably, the interior of the housing is made from a reflective material to assist in the application of UV light. In order to increase the automation of the apparatus, it may have an electronic control unit/central processing unit that controls the components of the apparatus. The components of the apparatus may include peristaltic pumps, platforms, the dispensing and removing member, the adjustable legs, sensors and associated components. Such an electronic control unit may be programmable. Preferably, the electronic control unit is an EPROM (erasable programmable read-only memory) device. The sensors may relay information to the electronic control unit and may additionally interface with a printer and/or a computer. Therefore, the apparatus can be re-programmed with a bespoke operating protocol in accordance with different laboratories requirements. Such reprogramming may be via a hand-held input device with a screen or alternatively may be via a computer linked to the apparatus. The computer may have a suitable user interface for ease of programming the apparatus and the computer may be connected to the apparatus by means of a standard cable such as a cable for connecting two USB ports together or similar (eg serial or parallel configurations) ports or connectors.

It will be apparent to the skilled addressee that the apparatus may be used for a number of different applications for processing biological material such as FISH, ISH, Reporter molecules, Immunostaining, Cytogenetics, High-throughput screening of compounds, cell culture, cell culture optimisation experiments etc. Furthermore, the apparatus may be used for processing the material in part of a protocol or indeed used for processing the whole sample when it is in a suitable format (such as placed on a slide for example). Preferably, the apparatus is used for processing biological material for cytogenetic analysis. Processing material for cytogenetic analysis may involve processing surface culture cells in order to analyze the associated chromosomes for abnormalities or for allelic variation etc. Commonly used techniques for cytogenetic analysis is via G/Q/R/T/C-banding and NOR silver staining, although other chromosome staining and banding techniques can also be utilized. It will apparent to those skilled in the art that the new techniques yet to be developed may also be used in conjunction with the present apparatus. Usually, the cells analyzed will be in cells driven to the metaphase cell cycle prior to processing, although cells in other stages e.g. interphase (FISH) may also be used.

When the apparatus is used to process the samples for chromosomal banding, the UV light source may be employed in addition to banding agents and by the addition and removal of respective reagents. Preferably, the use of the UV light source will be prior to the addition and removal of the reagents that are required for this technique. Reagents commonly used in chromosomal banding techniques, include hydrogen peroxide, trypsin, serum, dyes and buffers. Preferably, the apparatus mixes the hydrogen peroxide, the trypsin and the dye with their respective diluents in their required concentrations and quantities and are dispensed and removed by the apparatus depending on the required protocol. The apparatus may refrigerate some or all of the reagents so as to permit greater control over their reaction time with the sample. For example, fixatives may only be effective shortly after mixing and therefore the provision of refrigerating the fixative will allow the fixative to be used over a longer period of time. The apparatus can equally be used to process samples for FISH assays by the addition and removal of known reagents and probes. The dispensing member for such assays may need modification so that the precise quantity of probe used in the FISH assay can be controlled (due to the relative expense of such probes). Alternatively, the apparatus may have a dispensing member provided specifically for the addition of the probes, this may also be necessary for ISH, due to the requirement of radioisotopes.

The present invention will now be described by way of example only with reference to and as illustrated in the following figures;
Figure 1 is a perspective view of the apparatus in a closed position.
Figure 2 is a perspective view of the stacked platform within the apparatus.
Figure 3 is a perspective view of the apparatus in an open position.
Figure 4 is a exploded perspective view of the apparatus in an open position.
Figure 5 is a cross-sectional view of the dispensing and removing members.

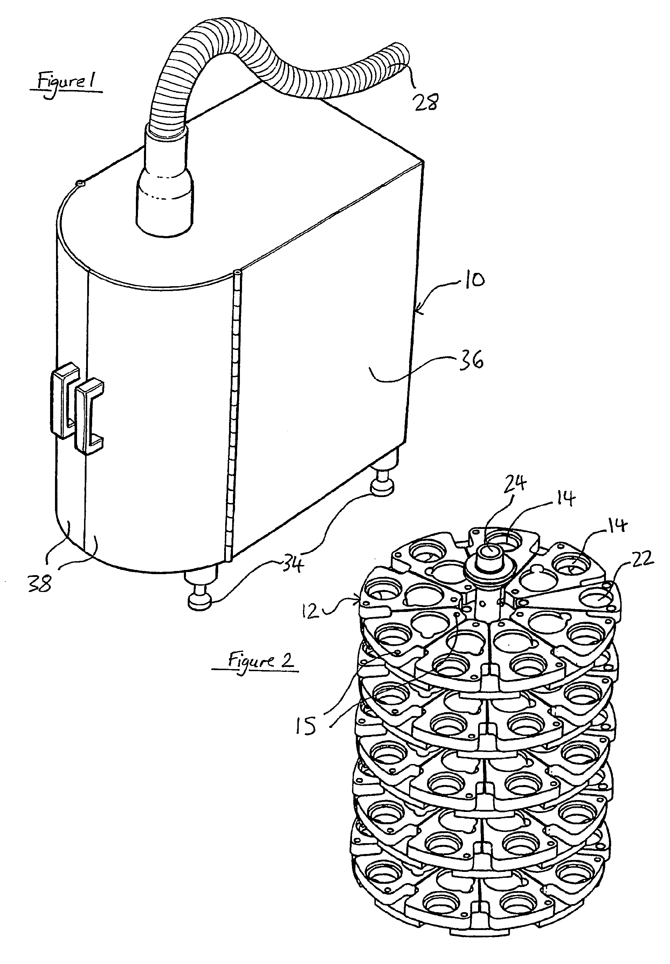

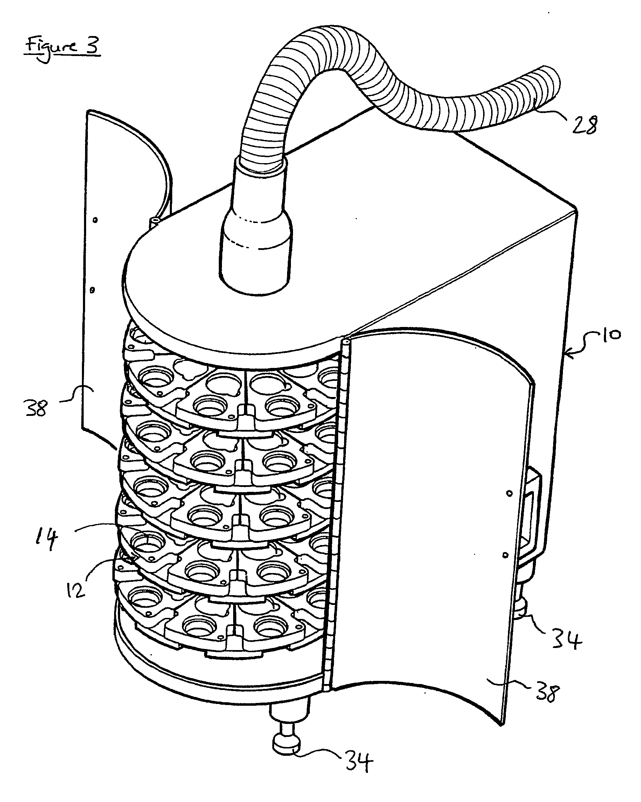

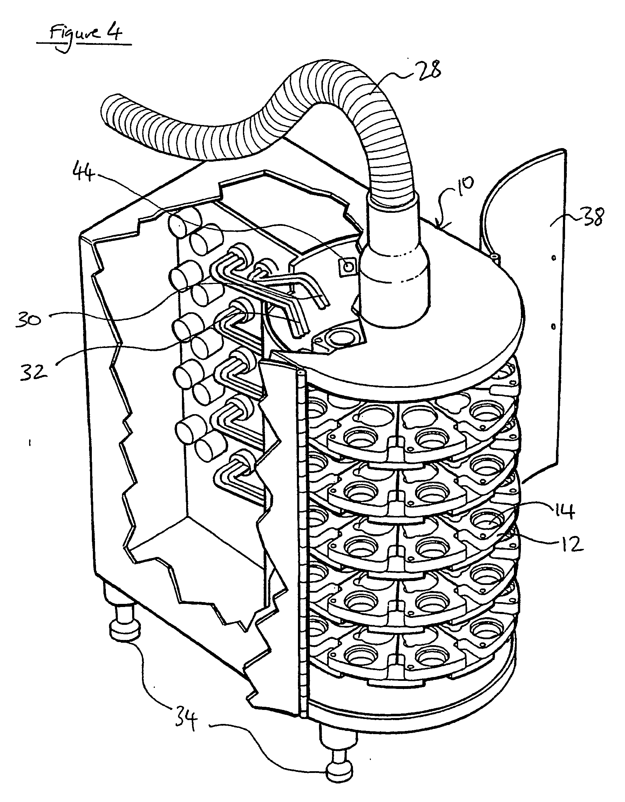

With reference to Figures 1 to 5, there is provided an apparatus 10 for processing biological material comprising five discs 12 which are capable of holding a number of samples of biological material which have been placed on a slide cover slip 13 and is placed on a 35mm petri dish 16. Each disc 12 has a number of sample receiving areas 14 which holds the petri dishes 16 in the correct position for processing by the apparatus 10. Each sample receiving area 14 is at an angle of approximately 3° relative to the disc 12 so that the petri dish 16 is inclined towards the periphery of the disc. There is also provided a lid holding area 18 on the disc 12 adjacent to the sample receiving area 14 which can hold a petri dish lid 20 whilst the biological material is being processed (as many laboratories print reference data on the lid). Each sample receiving area may be removable from the disc and replaced on locating pins 15 in order to allow the samples to be loaded away from the apparatus if needed. One part of the disc 12 has a wash station 22 for eluding excess reagents or waste. All the discs 12 are coupled to the central axis 24 which allows all the discs to rotate in unison. The central axis 24 moves in direction 26 by means of motor (not shown) which is electronically controlled. Each sample receiving area 14 on respective discs 12 are in the same position vertically. There is also provided an extraction fan (not shown) leading to an extraction tube 28 to remove fumes to the exterior of the apparatus. A number of dispensing and removal members 32 and 30 are situated in a vertical series in order for each disc sample receiving area 14 on each disc 12 can be serviced. A set of adjustable feet 34 are also provided in order to allow the apparatus to be placed on uneven or angled surfaces. The apparatus is contained within a housing 34 that has sealable doors 38 and also has a sensor 44 for detecting the presence of a sample in a sample receiving area 14.

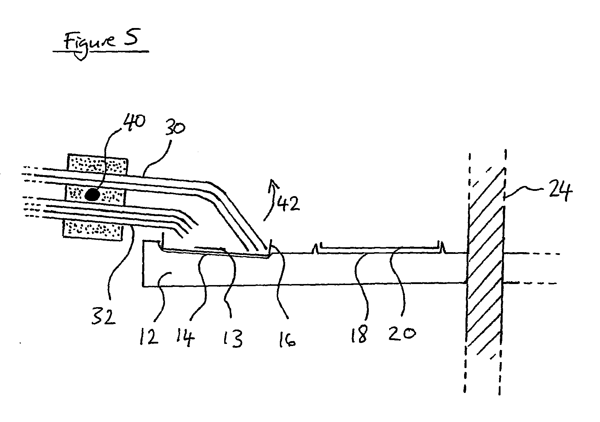

With reference to Figure 5, the dispensing member 32 has three dispensing tubes produced from PTFE tubing disposed within aluminium tubing. Each tube dispenses a different reagent. The removal member 30 has two removal tubes produced from a similar material to that of the dispensing tubes and allows for excess reagent and waste products after the reagent has been used to be removed from the petri dish 16. Two removal tubes are provided for the removal member in order to keep the apparatus operable should one tube become blocked. The removal tubes either rest directly onto the petri dish 16 or are very closely located near to the surface of the petri dish 16 in order to ensure maximum removal of waste material and liquid. The dispensing and removal tubes are operably joined to a pivot 40 in order that the tubes 30 and 32 can move vertically in direction 42 in order that it may allow the removal tube 30 to interface with a different sample on the same disc, when the disc 12 is rotated by means of a motor. The rocking action is linked to an electronic control unit (not shown) in order to initiate the rocking action when the disc movement is actuated.

The reagents are held in reagents vessels (not shown) into which supply tubes are inserted which are connected to peristaltic pumps that further supply the dispensing tubes 32 with reagent when required. The peristaltic pumps are also controlled electronically by the electronic control device. A waste vessel is also provided in order to receive waste material from the removal tubes, which are also connected to peristaltic pumps and controlled by the electronic control unit.

After the apparatus has been loaded with the number of samples to be processed, (the samples do not have to be loaded into sequential places if less than forty) the process can be initiated by starting the selected protocol (by selecting it on a keyboard linked to the electronic control unit). After the completed protocol has been applied to each sample the machine automatically stops, and if necessary alerts the operator. The estimated time for the completed cycle is approximately 1.5 - 2 hours for a typical protocol for cytogenetic cell processing.

Prior to the first or initial batch of samples, or at any other time between batches the complete system may be purged or rinsed out to the wash station 22 position. This facility will obviously be of use if a reagent vessel has been refilled or after an extended shutdown. The samples are loaded directly onto the disc or into the lift on/lift off sample receiving area and positioned on the discs 12 and the selected protocol initiated. The apparatus will automatically start by rotating the disc to No. 1 position and up to all 5 samples in that position are treated with the first reagent in the protocol. If the reaction time is very short the spent reagent could then be aspirated to the waste vessel by the removing tubes 30, the discs 12 then moving to position No. 2 to repeat the operation and running as necessary to position No. 8. The time required would normally be such that all additions would be completed before waste aspiration and dispersion of the second reagent. There are facilities for the sequential addition of up to six reagents although less or more than this may be used depending on the protocol that the apparatus is designed for. In some instances, two reagents may be supplied to the sample simultaneously, and subsequent removal of spent reagents as required. After the addition and removal of a particular reagent to and from all petri dishes 16, the apparatus automatically moves to the next step in the procedure, and in doing so runs through all stages required to complete the protocol. At completion the user is warned by a suitable signal (such as an audible tone or a light), and then the user manually unloads the dishes, containing the processed samples, prior, if necessary to reloading with the next batch of samples.

The apparatus is controlled by a built in program held in the electronic control device, which is re-programmable if necessary and such an electronic control device may have a number of protocols from which to choose from. The user may enter their own protocol as wished prior to a batch being processed, via the keyboard (not shown) which is linked to the electronic control unit. Alternatively, the electronic control unit may be linked to a computer via an interface so as to re-program the apparatus.

The apparatus can be used in a number of applications and one such application is in cytogenetic analysis. In this application, the apparatus is used in order to prepare surface cell culture driven to cell cycle metaphase in the cell cycle. Cells commonly used for cytogenetic analysis include fibroblast, placental mesodermal and amniocyte cells. The protocol was used to fix the cells prior to applying a process to highlight structural features within chromosomes, such as subjecting a protocol to G-Band the chromosomes). Forty samples can be loading onto the apparatus and they are then processed through the stages from adding a suitable hypotonic solution to the final fixing with acetic acid/methanol. After, the addition and reaction of each reagent there is the facility to remove, if necessary, the spent reagent before proceeding to the next stage of the process. Both the quantity of reagent and the reaction time will be controllable by the individual operator, although all samples in one loading of the machine will be subject to the same protocol and the facility for varying the protocol between loadings is provided by means of an input device or interface with a computer which can program the electronic control unit.

The apparatus is substantially leak proof, and in addition the main logic and control circuits together with most of the actuators are mounted in a compartment separate from the liquids providing for a reliable apparatus. In the unlikely event of a leak developing, it is not going to cause a major electrical or electronic fault. The electrical system inside the main module is entirely 12 and 24 volt, fed from a power supply mounted internally, and any fume extraction equipment is similarly organised and mounted separately from any equipment containing liquids.

The configuration of the apparatus ensures that all liquids are dispensed or removed by peristaltic pumps, which, provided scheduled maintenance is adhered to, are non-leaking. The pump delivery dose is controlled coarsely by tube size and pump rotational speed, the fine control being by programmed in relation to pump run time. A sensor 44, optical or magnetic or other detects the presence of a dish in any particular station, and only in the presence of a dish 12 does a pump dispense or remove liquid. When each operation, whether delivery or removal of liquid, at a particular dish, has been completed the dispensing tube 32 are lifted under control of the program, the disc 12 rotates to the next position that is controlled by a servomotor programmed in the required protocol, the tubes 30,32 are then lowered and the next step in the program initiated.

All liquids are stored and transferred in closed systems to reduce contamination (e.g. moisture pick up) as far as possible. The spent reagents are removed through a closed system to a waste vessel, sealed to the requirements of the user. The only position in which slides or similar cultures are exposed with reagents is in the dishes themselves and provision can be made for enclosing this volume if required. The enclosed space can be exhausted through a fume extraction system or could be subject to controlled atmospheric conditions.

Although based round a standard frame and operating procedure the exact configuration of the apparatus will be tailored to the users preferred protocol.

The machinery prefers a substantially level surface to stand on, a footprint of approximately 30x50 cms and a clear height of 60 cms, with a suitable 240 or 115 volts mains power point (although other voltages may also be employed to allow for the device to be used in different countries).

## Claims

1. An apparatus for processing biological materials, the apparatus comprising a plurality of stacked, rotatable platforms (12), each platform having a plurality of sample receiving areas (14) located thereon, the apparatus having a first dispensing member (30) mounted for dispensing reagent and/or a sample to a sample receiving area on a first stacked platform and a second dispensing member mounted for dispensing reagent and/or a sample to a sample receiving area on a second stacked platform, each platform being rotatable to move sequential sample receiving areas thereon into orientation with the mounted reagent dispensing member to receive reagent therefrom.

2. An apparatus as claimed in claim 1, wherein the apparatus further comprises a third dispensing member mounted for dispensing reagent and/or a sample to a sample receiving area on a third stacked platform.

3. An apparatus as claimed in either claim 1 or 2, wherein each stacked platform has an associated dispensing member mounted for dispensing reagent and/or a sample to a sample receiving area on each stacked platform.

4. An apparatus as claimed in any one of claims 1 to 3, wherein the apparatus further comprises a removing member for removing process waste from the sample receiving area.

5. An apparatus as claimed in any preceding claim, wherein the apparatus is substantially contained within a releasably sealed housing.

6. An apparatus as claimed in claim 5, wherein the housing is connected to an air extraction means.

7. An apparatus as claimed in any preceding claim, wherein the platforms are rotatable about a common central axis.

8. An apparatus as claimed in any preceding claim, wherein the platforms rotate in unison or independently.

9. An apparatus as claimed in any preceding claim, wherein the platforms are substantially horizontal and stacked above one another vertically.

10. An apparatus as claimed in any of claims 7 to 9, wherein the platform has one or more slots disposed from the edge of the platform to the centre of the platform to facilitate the removal of the platform from the axis.

11. An apparatus as claimed in any preceding claim, wherein the sample receiving area is angled relative to said platform in the range of 2° to 25° to allow liquid to collect at one part of sample.

12. An apparatus as claimed in any of claims 4 to 11, wherein the dispensing or removing member comprise one or more tubes

13. An apparatus as claimed in any of claims 4 to 12, wherein the dispensing and removing member can move vertically relative to the sample receiving area.

14. An apparatus as claimed in any one of claims 12 to 13, wherein separate tubes are provided to dispense different reagents.

15. An apparatus as claimed in any preceding claim, wherein the samples are held in or on one or more of the following holding apparatus: a Petri dish, a slide ,a slide cover slip or a culture chamber.

16. An apparatus as claimed in any preceding claim, wherein the samples have an identification means disposed thereon.

17. An apparatus as claimed in any preceding claim, wherein the apparatus further comprises a sensor for detecting the presence of a sample and/or identifying the sample.

18. An apparatus as claimed in claim 16 or claim 17, wherein the identification means is selected from one or more of the following:
a bar code, a dot code or a radio frequency and/or the sensor is selected from one or more of the following:
an optical sensor, a magnetic sensor, a laser scanner or a radio transmitter receiver.

19. An apparatus as claimed in any preceding claim, wherein the apparatus further comprises a sensor for detecting characteristics of the processed biological material.

20. An apparatus as claimed in any preceding claim, wherein the apparatus further comprises a UV light source.

21. An apparatus as claimed in any preceding claim, wherein the apparatus further comprises an air conditioning means.

22. An apparatus as claimed in any preceding claim, wherein the apparatus further comprises a turbidity monitor for assessing the turbidity of a sample.

23. An apparatus as claimed in any preceding claim, wherein an electronic control unit/central processing unit controls the components of the apparatus.

24. An apparatus as claimed in any preceding claim, wherein the apparatus is used for processing biological material for cytogenetic analysis.

25. An apparatus as claimed in any preceding claim, wherein the apparatus is used for processing surface culture cells in order to analyse the associated chromosomes.

## Patentansprüche

1. Vorrichtung zum Verarbeiten von biologischen Materialien, wobei die Vorrichtung eine Mehrzahl gestapelter, drehbarer Plattformen (12) aufweist, und jede Plattform eine Mehrzahl von darauf angeordneten Probeaumahmebereichen (14) aufweist, wobei die Vorrichtung ein erstes Ausgabeelement (30) aufweist, das zum Ausgeben von Reagenz und/oder einer Probe an einen Probenaufuahmebereich auf einer ersten gestapelten Plattform angebracht ist, und ein zweites Ausgabeelement aufweist, das zum Ausgeben von Reagenz und/oder einer Probe an einen Probeaufnahmebereich auf einer zweiten gestapelten Plattform angebracht ist, wobei jede Plattform drehbar ist, um aufeinanderfolgende Probeaufnahmebereiche darauf in Ausrichtung mit dem angebrachten Reagenzausgabeelement zu bringen und Reagenz von diesem zu erhalten.

2. Vorrichtung nach Anspruch 1, wobei die Vorrichtung weiter ein drittes Ausgabeelement aufweist, das zum Ausgeben von Reagenz und/oder einer Probe an einen Probeaufnahmebereich auf einer dritten gestapelten Plattform angebracht ist.

3. Vorrichtung nach einem der Ansprüche 1 oder 2, bei der jede gestapelte Plattform ein zugehöriges Ausgabeelement aufweist, das zum Ausgeben von Reagenz und/oder einer Probe an einen Probeaufnahmebereich auf jeder gestapelten Plattform angebracht ist.

4. Vorrichtung nach einem der Ansprüche 1 bis 3, wobei die Vorrichtung weiter ein Entfernungselement zum Entfernten von Prozessabfall aus dem Probeaufnahmebereich aufweist.

5. Vorrichtung nach einem vorhergehenden Anspruch, wobei die Vorrichtung im Wesentlichen in einem lösbar abgedichteten Gehäuse enthalten ist.

6. Vorrichtung nach Anspruch 5, bei der das Gehäuse an ein Luftabsaugmittel angeschlossen ist.

7. Vorrichtung nach einem vorhergehenden Anspruch, bei der die Plattformen um eine gemeinsame Mittelachse drehbar sind.

8. Vorrichtung nach einem vorhergehenden Anspruch, bei der sich die Plattformen gemeinsam oder unabhängig voneinander drehen.

9. Vorrichtung nach einem vorhergehenden Anspruch, bei der die Plattformen im Wesentlichen horizontal sind und vertikal übereinander gestapelt sind.

10. Vorrichtung nach einem der Ansprüche 7 bis 9, bei der die Plattform einen oder mehrere Schlitze enthält, die von der Kante der Plattform zur Mitte der Plattform verlaufen, um die Entfernung der Plattform von der Achse zu vereinfachen.

11. Vorrichtung nach einem vorhergehenden Anspruch, bei der der Probeaufnahmebereich in Bezug zu der Plattform im Bereich von 2° bis 25° abgewinkelt ist, damit sich Flüssigkeit an einem Teil der Probe sammeln kann.

12. Vorrichtung nach einem der Ansprüche 4 bis 11, bei der das Ausgabe- oder Entfernungselement ein oder mehrere Röhrchen aufweist.

13. Vorrichtung nach einem der Ansprüche 4 bis 12, bei der sich das Ausgabe- und Entfernungselement vertikal in Bezug zu dem Probeaufnahmebereich bewegen können.

14. Vorrichtung nach einem der Ansprüche 12 bis 13, bei der getrennte Röhrchen zum Ausgeben verschiedener Reagenzien vorgesehen sind.

15. Vorrichtung nach einem vorhergehenden Anspruch, bei der die Proben in oder auf einer oder mehreren der folgenden Halteeinrichtungen gehalten werden: einer Petrischale, einem Objektträger, einem Objektträger-Deckglas oder einer Kulturkammer.

16. Vorrichtung nach einem vorhergehenden Anspruch, bei der die Proben ein auf denselben angeordnetes Identifikationsmittel aufweisen.

17. Vorrichtung nach einem vorhergehenden Anspruch, wobei die Vorrichtung weiter einen Sensor zum Ermitteln der Anwesenheit einer Probe und/oder zum Identifizieren der Probe aufweist.

18. Vorrichtung nach Anspruch 16 oder Anspruch 17, bei der das Identifikationsmittel von einem oder mehreren der Folgenden ausgewählt wird:
einem Strichcode, einem Punktcode oder einer Funkfrequenz und/oder der Sensor von einem oder mehreren der Folgenden ausgewählt wird:
einem optischen Sensor, einem Magnetsensor, einem Laserscanner oder einem Funk-Sender/ Empfänger.

19. Vorrichtung nach einem vorhergehenden Anspruch, wobei die Vorrichtung weiter einen Sensor zum Ermitteln der Eigenschaften des verarbeiteten biologischen Materials aufweist.

20. Vorrichtung nach einem vorhergehenden Anspruch, wobei die Vorrichtung weiter eine UV-Lichtquelle aufweist.

21. Vorrichtung nach einem vorhergehenden Anspruch, wobei die Vorrichtung weiter ein Klimagerät aufweist.

22. Vorrichtung nach einem vorhergehenden Anspruch, wobei die Vorrichtung weiter einen Trübungswächter zum Bewerten der Trübung einer Probe aufweist.

23. Vorrichtung nach einem vorhergehenden Anspruch, bei der eine elektronische Steuereinheit/Zentraleinheit die Komponenten der Vorrichtung steuert.

24. Vorrichtung nach einem vorhergehenden Anspruch, wobei die Vorrichtung zum Verarbeiten von biologischem Material für zytogenetische Analyse verwendet wird.

25. Vorrichtung nach einem vorhergehenden Anspruch, wobei die Vorrichtung zum Verarbeiten von Oberflächen-Kulturzellen verwendet wird, um die zugehörigen Chromosomen zu analysieren.

## Revendications

1. Appareil de traitement de produits biologiques, l'appareil comprenant une pluralité de plates-formes rotatives empilées (12), chaque plate-forme étant munie d'une pluralité de zones de réception des échantillons (14) situées dessus, l'appareil étant muni d'un premier organe de distribution (30) installé pour distribuer un réactif et/ou un échantillon à une zone de réception des échantillons sur une première plate-forme empilée et un deuxième organe de distribution installé pour distribuer un réactif et/ou un échantillon à une zone de réception des échantillons sur une deuxième plate-forme empilée, chaque plate-forme étant rotative pour déplacer successivement les zones de réception des échantillons successives se trouvant sur elle en les orientant avec l'organe de distribution du réactif installé afin d'en recevoir du réactif.

2. Appareil conforme à la revendication 1, où l'appareil comporte de plus un troisième organe de distribution installé pour distribuer du réactif et/ou un échantillon dans une zone de réception des échantillons sur une troisième plate-forme empilée.

3. Appareil conforme à la revendication 1 ou 2, où chaque plate-forme empilée est munie d'un organe de distribution associé installé pour distribuer du réactif et/ou un échantillon dans une zone de réception des échantillons sur chaque plate-forme empilée.

4. Appareil conforme à une quelconque des revendications 1 à 3, où l'appareil comporte de plus un organe de retrait pour retirer les déchets de procédé de la zone de réception des échantillons.

5. Appareil conforme à une quelconque des revendications précédentes, où l'appareil est essentiellement contenu à l'intérieur d'un logement scellé provisoirement.

6. Appareil conforme à la revendication 5, où le logement est connecté à un moyen d'extraction d'air.

7. Appareil conforme à une quelconque des revendications précédentes, où les plates-formes sont rotatives autour d'un axe central commun.

8. Appareil conforme à une quelconque des revendications précédentes, où les plates-formes tournent à l'unisson ou séparément.

9. Appareil conforme à une quelconque des revendications précédentes, où les plates-formes sont sensiblement horizontales et empilées verticalement l'une au-dessus de l'autre.

10. Appareil conforme à une quelconque des revendications 7 à 9, où la plate-forme présente une ou plusieurs fentes disposées depuis le bord de la plate-forme jusqu'au centre de la plate-forme pour faciliter le retrait de la plate-forme de l'axe.

11. Appareil conforme à une quelconque des revendications précédentes, où la zone de réception des échantillons est inclinée par rapport à ladite plate-forme dans une plage allant de 2° à 25° pour permettre au liquide de s'accumuler à une partie de l'échantillon.

12. Appareil conforme à une quelconque des revendications 4 à 11, où l'organe de distribution ou de retrait comprend un ou plusieurs tubes.

13. Appareil conforme à une quelconque des revendications 4 à 12, où les organes de distribution et de retrait peuvent se déplacer verticalement par rapport à la zone de réception des échantillons.

14. Appareil conforme à une quelconque des revendications 12 à 13, où des tubes séparés sont prévus pour distribuer différentes réactifs.

15. Appareil conforme à une quelconque des revendications précédentes, où les échantillons sont tenus dans ou sur un ou plusieurs des appareils de retenue suivants : boîte de Pétri, lame, lamelle couvre-objet ou chambre de culture.

16. Appareil conforme à une quelconque des revendications précédentes, où les échantillons portent un moyen d'identification.

17. Appareil conforme à une quelconque des revendications précédentes, où l'appareil comprend de plus un capteur pour détecter la présence d'un échantillon et/ou identifier l'échantillon.

18. Appareil conforme à la revendication 16 ou 17, où le moyen d'identification est sélectionné parmi l'un ou plusieurs des suivants :
un code barres, un code points ou une fréquence radio et/ou le capteur est sélectionné parmi l'un ou plusieurs des suivants :
capteur optique, capteur magnétique, lecteur laser ou émetteur-récepteur radio.

19. Appareil conforme à une quelconque des revendications précédentes, où l'appareil comporte en outre un capteur pour détecter des caractéristiques du produit biologique traité.

20. Appareil conforme à une quelconque des revendications précédentes, où l'appareil comporte en outre une source de lumière UV.

21. Appareil conforme à une quelconque des revendications précédentes, où l'appareil comporte en outre un moyen de climatisation.

22. Appareil conforme à une quelconque des revendications précédentes, où l'appareil comporte en outre un appareil de mesure de la turbidité pour évaluer la turbidité d'un échantillon.

23. Appareil conforme à une quelconque des revendications précédentes, où une unité de commande électronique/unité centrale de traitement commande les composants de l'appareil.

24. Appareil conforme à une quelconque des revendications précédentes, où l'appareil sert à traiter un produit biologique pour une analyse cytogénétique.

25. Appareil conforme à une quelconque des revendications précédentes, où l'appareil sert à traiter des cellules en culture de surface afin d'analyser les chromosomes associés.
